# EUROPEAN PATENT SPECIFICATION

(11) **EP 1 506 316 B1**
(45) Date of publication and mention of the grant of the patent: **16.01.2008**
(21) Application number: 03732887.9
(22) Date of filing: 21.05.2003
(51) Int. Cl.: C12Q 1/68

(54) **METHOD FOR IMPROVING EFFICIENCIES IN LIVESTOCK PRODUCTION**
VERFAHREN ZUR VERBESSERUNG DER EFFIZIENZ DER TIERZÜCHTUNG
PROCEDE DESTINE A ACCROITRE LES RENDEMENTS DANS L'INDUSTRIE DES PRODUCTIONS ANIMALES

(30) Priority: 21.05.2002 CA 2387003
(43) Date of publication of application: 16.02.2005
(73) Proprietor: Marquess, Foley Leigh Shaw, Alberta, Toronto TOJ 1MO (CA)
(72) Inventor: Marquess, Foley Leigh Shaw, Alberta, Toronto TOJ 1MO (CA)
(74) Representative: Williams, Aylsa
(86) International application number: PCT/IB2003/002575
(87) International publication number: WO 2003/097876

(56) References cited:
- WO-A-00/06777
- WO-A-02/20850
- BUCHANAN FIONA C ET AL: "Association of a missense mutation in the bovine leptin gene with carcass fat content and leptin mRNA levels" GENETICS SELECTION EVOLUTION (PARIS), vol. 34, no. 1, January 2002 (2002-01), pages 105-116, XP008023609 ISSN: 0999-193X cited in the application
- ZWIERZCHOWSKI LECH ET AL: "An association of growth hormone, kappa-casein, beta-lactoglobulin, leptin and Pit-1 loci polymorphism with growth rate and carcass traits in beef cattle" ANIMAL SCIENCE PAPERS AND REPORTS, vol. 19, no. 1, 2001, pages 65-77, XP001127452 ISSN: 0860-4037
- KLAUZINSKA M ET AL: "COMPARISON OF SELECTED GENE POLYMORPHISMS IN POLISH RED AND POLISH BLACK-AND-WHITE CATTLE" ANIMAL SCIENCE PAPERS AND REPORTS, POLISH SCIENTIFIC PUBLISHERS, WARZSAW, PL, vol. 18, no. 2, 2000, pages 107-116, XP001127451 ISSN: 0860-4037

## Description

### RELATED APPLICATIONS

This application claims priority to Canadian Application No. 2,387,003, entitled: "METHOD FOR IMPROVING EFFICIENCIES IN LIVESTOCK PRODUCTION", filed May 21, 2002.

### FIELD OF THE INVENTION

The present invention relates to a method of managing livestock animals according to their genotypes and, more specifically, is directed to a method of managing livestock in groups having predictably more uniform fat deposition than is presently possible.

### BACKGROUND OF THE INVENTION

Leptin and the *ob* Gene: Leptin, a 16-kDa adipocyte-specific polypeptide is expressed predominantly in fat tissues of those animals in which it has been detected, which animals include livestock species such as cattle, pigs, and sheep. Leptin is encoded by the *ob* (obese) gene and appears to be involved in the regulation of appetite, basal metabolism and fat deposition. Increased plasma concentrations of leptin in mice, cattle, pigs and sheep have been associated with decreased body fat deposition and appetite, and increased basal metabolism levels (Blache et al., 2000; Delavaud et al., 2000; Ehrhardt et al., 2000). Similar phenotypic characteristics have also been found to be associated with leptin mRNA levels in adipose tissue (Ramsay et al., 1998; Robert et al., 1998). Consistent with those observations, it has been shown that administration of exogenous leptin dramatically reduces feed intake and body mass of mice, chickens, pigs and sheep (Barb et al.,1998; Halaas et al., 1995; Henry et al., 1999; and Raver et al., 1998).

The ob gene that has been mapped to chromosome 6 in mice (Friedman and Leibel, 1992), chromosome 7q31.3 in humans (Isse et al., 1995) chromosome 4 in cattle (Stone et al. 1996), and chromosome 18 in swine (Neuenschwander et al., 1996; Saskai et al., 1996). Sequences have been determined for the said gene from mice (Zhang et al., 1994), cattle (U.S. Patent No. 6,297,027 to Spurlock), pigs (U.S. Patent No. 6,277,592 to Bidwell and Spurlock; Neuenschwander et al., 1996), and humans (U.S. Patent No. 6,309,857 to Friedman et al.) and there is significant conservation among the sequences of *ob* DNAs and leptin polypeptides from those species (Bidwell et al. 1997; Ramsay et al. 1998).

Mutations in the coding sequences of the ob gene causing alterations in the amino acid sequence of the leptin polypeptide, have been associated with hyperphagia, hypometabolic activity, and excessive fat deposition; i.e., a phenotype characterized by larger body size; a fat phenotype (Zhang et al., 1994).
ob-Gene Genotypes: Fitzsimmons et al., (1998) reported evidence of three alleles of a micosatellite marker located proximal to the ob gene in cattle that occurred with significant frequency in bulls of several breeds (Angus, Charolais, Hereford and Simmental) and comprising 138, 147 and 149 base pairs (bp). The 138-bp and 147-bp alleles, respectively, occurred most frequently. Further, it was determined that occurrence of the 138-bp allele was positively associated with certain carcass characteristics; increased average fat deposition, increased mean fat deposition, increased percent rib fat, and decreased percent rib lean. Thus, bulls homozygous for the 138-bp allele exhibited greater average fat deposition than heterozygous animals and such heterozygotes exhibited greater average fat deposition that bulls homozygous for the 147-bp allele.

Subsequently, Buchanan et al. (2002) identified a cytosine (C) to thymine (T) transition within an exon (exon 2) of the *ob* gene, corresponding to an arginine (ARG) to cysteine (CYS) substitution in the leptin polypeptide. The presence of the T-containing allele in bulls was associated with fatter carcasses than those from bulls with the C-containing allele.

Single nucleotide polymorphisms have also been detected in the porcine ob gene and certain of those polymorphisms have been found to be associated with feed intake and carcass traits (Kennes et al. 2001; Kulig et al. 2001).
*ob*-Gene Genotype Determination: Means of selective amplification of bovine gene are in U.S. patent No. 6,297,027 to Spurlock. It is possible to distinguish ob genotypes by cloning and sequencing DNA fragments from individual animals, or by other methods known in the art. For example, it is possible to distinguish ob genotypes by employing synthetic oligonucleotide primed amplification of *ob* gene fragments followed by restriction endonuclease digestion of the amplified product using a restriction enzyme that cuts such product from different ob alleles into discrete product fragments of differing length. Such discrete product fragments could then be distinguished using electrophoresis in agarose or acrylaminde, for example. The ob alleles identified by Buchanan et al. (2002) were distinguished by such means using a mismatch PCR-RFLP strategy wherein, the C-containing allele (as above) yields DNA fragments of 75 and 19 bp following digestion of the amplimer with Kpn 21, and the T-containing allele (as above) is not cut.
The Development of Desired Body Condition in Livestock Animals

Body condition is a determinant of market readiness in commercial livestock feeding and finishing operations. The term body condition is used in livestock industry in reference to the state of development of a livestock animal that is a function of frame type or size, and the amount of intramuscular fat and back fat exhibited by an animal. It is typically determined subjectively and through experienced visual appraisal of live animals. The fat deposition, or the amount of intramuscular fat and back fat on an animal carcass, is important to industry participants because carcasses exhibiting desired amounts and proportions of such fats can often be sold for higher prices than carcasses that exhibit divergences from such desired amounts and proportions. Furthermore, the desired carcass fat deposition often varies among different markets and buyers, and also often varies with time in single markets and among particular buyers in response to public demand trends with respect to desired of fat and marbling in meat. Weight gain by a livestock animal during its growth and development typically follows a tri-phasic pattern that is carefully managed by commercial producers, and finishers. The efficiency of dietary caloric (feed) conversion to weight gain during an increment of time varies during three growth phases; a first phase of growth comprises that portion of a livestock animals life from birth to weaning, and is not paid much heed by commercial feeding and finishing operators.

A second growth phase comprises that portion of a livestock animal's life from weaning to attainment of musculo-skeletal maturity. Feed conversation efficiency is low during this phase; livestock producers usually restrict caloric intake, which has the effect of causing this phase to be prolonged but also typically results in animals with larger frames, which is the aim of dietary management during this phase. During the second growth phase weight gain is associated with skeletal mass and muscle mass accumulation primarily.

During a third growth phase, after a animal has attained musculo-skeletal maturity, the efficiency of feed conversion is reduced, such that it requires more feed to increase an animal's weight. For example with cattle, during the second phase of growth, a typical steer could convert 5 to 6 pounds of feed into one pound of weight gain. Upon entering the third phase, feed conversion efficiency typically decreases, such that 7 up to 10 or more pounds of feed are required to produce one pound of gain. During the third phase livestock feeders significantly increase the caloric content of animals' rations. During the third growth phase weight gain is associated with fat accumulation primarily. Again using cattle as an example, with a steer weighing 900 pounds at the end of the second phase, of that 900 pounds, typically 350 pounds will be red meat. At the end of the third phase, the steer would typically weigh 1400 pounds and typically 430 pounds will be red meat.

Keeping the cattle industry as an example, initially a cow/calf operator will breed bulls to cows, birth calves from the cows, and allow the calves to feed on their mother's milk until they are weaned some months after birth. This is the first phase of growth of the calf. After weaning, the calf enters the second stage of growth where it is fed to grow to its full skeletal size. This commonly called the "backgrounding" phase during which musculo-skeletal maturity is achieved.

When the animal has reached its full size, it enters the third phase of growth where the fully grown animal puts on weight. Typically it is at the start of the third stage of growth that the animal enters a finishing feed lot. In the feed lot the *ob*ject is to feed the animal the proper ration so that it will most quickly obtain the proper market characteristics that are desired at that given time. At present, for instance it is desirable to have beef that is well marbled, *ie* it has considerable intramuscular fat in the meat. At other times it may be desirable to have lean meat with very little intramuscular fat. The price the feed lot owner attains for his cattle, when he sells to the packer can vary significantly depending on marbling of the meat.

Presently, cattle entering a feed lot are divided into groups according to estimated age, frame size, breed, weight and so forth. By doing this the feed lot owner is attempting to group the cattle so that the group can be penned together and fed the same ration and will be ready for market at the same time. Weight and visual clues are the only means possible to sort cattle for feed lot grouping.

Once the cattle are sold from the feed lot to the packer they are slaughtered and the carcasses are hung on a rail where they can be graded according to the amount of fat measured at certain defined and standardized points on the carcass. This fat measurement is accepted as correlating to the amount of intramuscular fat in the carcass. A carcass with a fat measurement at or above a certain standard measurement will be graded AAA in Canada, corresponding to Choice Grade in the United States. A carcass with a fat measurement less than that set for AAA grade, but above the standard set for AA grade, will grade AA, while those with fat measurements below the standard set for AA be graded correspondingly lower through the range of grades.

The most desirable grade in the present market is AAA, because fat is equated with palatability, lending juiciness and tenderness to the meat, and is presently seeing demand from consumers. Significant premiums are presently being paid for carcasses grading AAA. In contrast, premiums have been historically been seen for leaner beef. At any given time then, the consumer will indicate his preference at the retail shelf, and this will send signals back through the chain to the packer, feeder, and cow/calf operators to aim for more or less fat.

Conventionally, the chain has reacted to these signals by switching breeds. Broadly speaking, European breeds such as Charolais and Limousin have bigger frames and leaner meat than British breeds such as Hereford and Angus. When lean beef is in demand, the feed lot will pay premiums for cattle bearing traits of European breeds, and when fat beef is in demand, premiums are paid for cattle bearing traits of British breeds.

Another major factor in the price realized by the feed lot operator is the yield grade, which is the percentage of usable meat that is derived from a carcass. Yield grade is dictated by a maximum fat measurement, but is a grade that is independent of the palatability' grade. While the minimum fat measurement for AAA grade may be achieved, exceeding that measurement can cause a reduction in yield grade, and therefore a reduction in price. For each yield grade there is a maximum fat measurement, such that exceeding a maximum fat measurement for Yield Grade 1 drops the carcass to a Yield Grade 2, and exceeding a maximum fat measurement for Yield Grade 2 drops the carcass to a Yield Grade 3, and so forth. Essentially the yield grade accounts for excessive fat on the carcass that must be trimmed prior to sale, and is therefore waste.

Thus to realize the maximum price for a carcass in a market like that at present where the AAA grade is in demand, the feed lot operator must meet the minimum fat measurement for AAA grade, and yet not exceed the maximum fat measurement for Yield Grade 1. Present methods used to achieve this goal comprise visually grouping cattle according to frame type, estimated age and estimated weight at the time the cattle enter the feed lot. The animals of a particular group are fed and otherwise maintained substantially uniformly until it is estimated, again on the basis of experienced visual inspection, that the mean body condition of animals in the group is such that the measurement of fat will exceed the minimum required for AAA grade, yet be below the maximum allowed for Yield Grade 1.

In addition to palatability and yield grades, other factors also influence the price received for a carcass. For example the weight of the carcass should fall in a desired range that provides the most popular size of cuts of meat.

Regardless of the particular market preference at any given time, the feed lot operator will be trying to tailor his cattle to meet some similar standard that will cause a meat packer or like commercial purchaser to pay the highest price in accordance with currently prevailing market preferences.

Invariably some carcasses from the animals in a group fall in the desired range, while many are outside the desired range. Thus some of the carcasses w ill bring the maximum price because they are in the desired range, but a great many will bring a reduced price because they are outside the desired range. The price reduction generally increases in steps as variation from the desired range increases.

The feed lot operator's costs include the costs of operating the feed lot, such as labor, capital, maintenance, etc., plus the cost of feeding the cattle. While the cost of acquiring each animal in a group can vary somewhat, the feed lot operator's costs are the s ame f or e ach animal in the group since they are fed the same amount of feed and occupy space in the feed lot for the same amount of time. Thus the price reductions for carcasses falling outside the desirable range fall directly to the feed lot operator's bottom line, reducing profits.

The feed lot operator has a very complex set of factors to consider when making decisions regarding feeding and marketing cattle. The longer the animal is in the feed lot before sale, the more it has cost the feed lot operator. At some times, keeping animals longer might be an attractive option if by doing so a more profitable grade can be achieved. For instance when body fat is in demand, the feed lot might keep the animals longer to fatten them more in order to have more cattle reach the AAA, grade. This is especially true where yield grade deductions for excess fat are less than premiums for sufficient fat, and even more so at times when sufficient animals are not available to bring into the feed lot, or when the price for same is high. The variability in the propensity of cattle to accumulate fat significantly reduces the efficiency and profitability of feed lots.

Presently packers predict the carcass grade of the animals they buy based on visual clues and experience. Packers take orders for assorted quantities of AAA and other grades of beef which they must then fill from the cattle that they buy from feed lots. The grading mix of these animals can vary considerably and thus the packer faces considerable difficulty in predicting what his supply of the various grades of carcasses will be at any given time. The packer is often required to go out and buy on short notice more cattle to a fill an order for a particular grade, again basing his decision on which cattle to buy on visual clues as to how the carcass will grade when it is finally hanging on the rail in his plant.

After cattle are slaughtered, the carcasses are brought into a cooler where they hang for 20 or more hours prior to grading to allow a proper fat measurement to be taken. Once graded the carcasses are left to hang for typically 14- 21 days. The cooler thus contains, at any given time, a considerable number of un-graded carcasses. As the carcasses are graded the packer must continually assess his inventory against his orders, and then buy cattle appropriately. Depending on the inventory and orders, a packer will typically be seeking to buy fatter or leaner cattle. A surplus of one or the other will typically require a price reduction in order to move the surplus out of the cooler on a timely basis. Such price reductions reduce the packer's profits. Increased accuracy in predicting the carcass grade of cattle purchased would reduce the occurrence of surpluses, and increase the packer's profit.

As discussed above, cow/calf operators breed bulls to cows, choosing the mating based on signals received through the chain of supply from consumers for those traits that are in demand, for example fat beef or lean beef. European breeds provide carcasses that are typically leaner than British breeds, therefore the cow/calf operator will typically lean to one or the other as demand changes. They also select breeding animals based on visual traits, such as frame size, and anectodal traits, such as easy calving history. Again, the object is to provide cattle that will command the highest price from the eventual purchaser, such a backgrounder or feed lot operator.

### SUMMARY OF THE INVENTION

It is the *ob*ject of the present invention to provide a method for improving efficiencies in livestock production. In one embodiment of the present invention such a method comprises grouping livestock animals, such as cattle and pigs, during the period of their retention in a feeding facility according to the genotype of individual livestock animals to deposit fat, and then feeding the animals in each group substantially uniformly.

It is a further object of the present invention to provide a method comprising meeting particular body fat acquisition expectations. In one embodiment, homozygosity or heterozygosity of e ach animal i s determined with respect t o alleles of a gene encoding an adipocyte-specific polypeptide, termed leptin, which gene is hereinafter referred to as ob, and segregating such animals into groups based on genotype, e.g., ob genotype, and optionally, phenotype. In one embodiment, animals are segregated by phenotype, e.g., frame type and genotype, *e.g.,* homozygosity in respect of a first *ob* allele, homozygosity in respect of a second ob allele, or heterozygosity in respect of the first and second *ob* alleles. The feeding and otherwise maintaining animals in a group together and apart from other groups of animals, and ceasing to feed the animals in the group at a time is sustained until the median body fat condition of the animals of that group is of a desired body fat condition.

Yet another embodiment, the present invention provides a method of managing cattle entering a feed lot, by determining homozygosity or heterozygosity of animals with respect to alleles of the ob gene, and sorting the cattle accordingly into three groups, one group homozygous in respect of a first *ob* allele and therefore having the most propensity to deposit fat, a second group homozygous in respect of a second ob allele and therefore having the least propensity to deposit fat, and a third group heterozygous in respect of the first and second ob alleles and therefore having an intermediate propensity to deposit fat. It is a further object of the present invention to provide such a method wherein the three groups are further divided according to weight or frame size.

It is a further object of the present invention to provide a method comprising, for groups of animals having the least genetic predisposition to produce fat, feeding to achieve an animal carcass having a low median body fat.

A further embodiment of the p resent invention to provides a method to packers t o increase predictability of the fat deposition in groups of livestock purchased. In particular, this embodiment allows cow/calf operators to respond to market signals from the feed lot more accurately by producing animals having greater or lesser genetic predisposition to lay down fat.

In the method of the present invention, individual animals, among assemblies of animals received at feeding facilities, are segregated into groups based conventionally on weight and frame type, and additionally based on ob genotype. Preferably and most efficiently the animals are tested to determine homozygosity or heterozygosity with respect to alleles of the ob gene as they are received at the receiving facility, and are grouped accordingly with little interruption in the normal flow of animals through the facility. Animals of such groups will, when maintained together on a uniform diet, exhibit greater body fat condition uniformity at any particular time after such segregation than is exhibited among animals grouped together using current practices.

Individual animals within such a group will attain a desired body condition closer to the time that other individual animals of the same group attain the desired body condition. Such temporal uniformity exceeds that exhibited in groups of otherwise similarly situated animals maintained and fed together using current grouping practices.

It will be advantageous to feed cattle to achieve a high fat grade when they are most genetically predisposed to lay down fat (hereafter TT cattle, i.e., cattle homozygous for the T SNP). As to those cattle least genetically predisposed to lay down fat (hereafter CC cattle, i.e., homozygous for the C SNP), it will be advantageous to feed these cattle so as to achieve a lower fat grade, or a lean grade, rather than feed them longer to achieve the high fat grade. Those cattle intermediately genetically predisposed to lay down fat, (hereafter CT cattle, i.e. heterozygous for the SNP), can be fed longer to achieve a high fat grade, or shorter to achieve a lean grade, depending on considerations such as market prices, price trends, feed costs, availability of further feeder cattle to bring into the feed lot, and other like external considerations. On occasion such external considerations may dictate that CC cattle should be fed for a fat grade, however this will most often be so inefficient that such feeding would not be cost effective.

A further advantage of feeding CC cattle for a lean grade would be realized by the packer who buys the cattle. Packers receive orders for fat beef and lean beef. Presently packers faced with an order for fat AAA beef are very often forced to buy considerably more cattle than they actually need in order to ensure that they have sufficient high fat AAA carcasses to meet the order. They thus have an excess of lean AA or A grade beef that they sell off at reduced prices. If a packer was confident that when buying a certain number of market ready TT cattle, he would get 55% - 65% AAA grade, then he could fill the AAA grade order with less cattle, and properly fill his lean AA beef requirements from CT or CC animals fed to the leaner grade. CT cattle would be somewhat more mixed, however it is foreseen that CC cattle could be fed efficiently such that 80% or more would grade lean.

It is noted that in this disclosure and particularly in the claims, terms such as "comprises", "comprised", "comprising" and the like can have the meaning attributed to it in U.S. Patent law; e.g., they can mean "includes", "included", "including", and the like; and that terms such as "consisting essentially of' and "consists essentially of' have the meaning ascribed to them in U.S. Patent law, e.g., they allow for elements not explicitly recited, but exclude elements that are found in the prior art or that affect a basic or novel characteristic of the invention.

These and other objects, features, and advantages of the invention become further apparent in the following detailed description of the invention when taken in conjunction with the accompanying drawings and claims which illustrate, by way of example, the principles of this invention.

### BRIEF DESCRIPTION OF THE DRAWING

A full and enabling disclosure of the present invention, including the best mode thereof, to one of ordinary skill in the art, is set forth more particularly in the remainder of the specification, including reference to the accompanying figures, wherein:

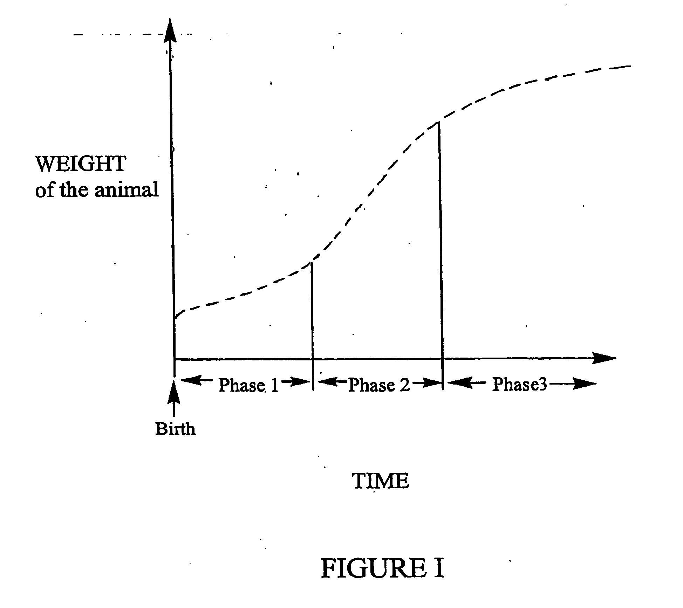

Fig. 1 illustrates the growth curve of production animals, such as poultry, pigs, sheep, and cattle, wherein the phase of growth is correlated with the weight of the animal.

### DETAILED DESCRIPTION

Other objects, features and aspects of the present invention are disclosed in, or are *ob*vious from, the following Detailed Description. It is to be understood by one of ordinary skill in the art that the present discussion is a description of exemplary embodiments only and is not intended as limiting the broader aspects of the present invention, which broader aspects are embodied in the exemplary construction. In fact, it will be apparent to those skilled in the art that various modifications and variations can be made in the present invention without departing from the scope or spirit of the invention. For instance, features illustrated or described as part of one embodiment can be used in another embodiment to yield a still further embodiment. It is intended that the present invention cover such modifications and variations as come within the scope of the appended claims and their equivalents.

For convenience, certain terms employed in the Specification, Examples, and appended Claims are collected herein as follows:

The term "animal" is used herein to include all vertebrate animals, excluding humans. It also includes an individual animal in all stages of development, including embryonic and fetal stages.

As used herein, the term "production animals" is used interchangeably with "livestock animals" and refers generally to animals raised primarily for food. For example, such animals include, but are not limited to, cattle (bovine), sheep (ovine), pigs (porcine or swine), poultry (avian), and the like.

As used herein, the term "cow" or "cattle" is used generally to refer to an animal of bovine origin of any age. Interchangeable terms include "bovine", "calf", "steer", "bull", "heifer" and the like.

The term "avian" as used herein refers to any species, subspecies or race of organism of the taxonomic class ava, such as, but not limited to, such organisms as chicken, turkey, duck, goose, quail, pheasants, parrots, finches, hawks, crows and ratites including ostrich, emu and cassowary.

As used herein, the term "pig" or is used generally to refer to an animal of porcine origin of any age. Interchangeable terms include "piglet", "sow" and the like.

As used herein, the term "Genome" refers to all the genetic material in the chromosomes of a particular organism. Its size is generally given as its total number of base pairs. Within the genome, the term "gene" refers to an ordered sequence of nucleotides located in a particular position on a particular chromosome that encodes specific functional product (e.g., a protein or RNA molecule). For example, it is known that the protein leptin is encoded by the *ob* (obese) gene and appears to be involved in the regulation of appetite, basal metabolism and fat deposition In general, an animal's genetic characteristics, as defined by the nucleotide sequence of its genome, are known as its "genotype, while the animal's physical traits are described as its "phenotype."

As used herein, the term "locus" or "loci" refers to the site of a gene on a chromosome. Pairs of genes, also known as "alleles" control the hereditary traits, each in the same position on a pair of chromosomes. These alleles, which also may be described as an animal's "allelotype" may both be dominant or recessive in expression of that trait. In either case, the individual is said to be homozygous for the trait controlled by that gene pair. If the gene pair (alleles) consists of one dominant and one recessive trait, the individual is heterozygous for the trait controlled by the gene pair.

The term "Nucleotide" generally refers to a subunit of DNA or RNA consisting of a nitrogenous base (adenine, guanine, thymine or cytosine in DNA; adenine, guanine, uracil, or cytosine in RNA) a phosphate molecule, and a sugar molecule (deoxyribose in DNA and ribose in RNA). Thousands of nucleotides are linked to form a NDA or RNA molecule. A "Single Nucleotide Polymorphism" or SNP is used herein to refer to the most common type of genetic variation in a gene consisting of a change at a single base in a DNA molecule. One example of a SNP is the cytosine (C) to thymine (T) transition within exon 2 of the ob gene, corresponding to an arginine (ARG) to cysteine (CYS) substitution in the leptin polypeptide (Buchanan et al. (2002).

As used herein, the term "Protein" generally refers to a large molecule composed of one or more chains of amino acids in a specific order. The order is determined by the base sequence of nucleotides in the gene coding for the protein. Proteins are required for the structure, function, and regulation of the body's cells, tissues, and organs. Each protein has a unique function.

A typical growth curve for production animals is illustrated in Figure 1. Present production practices vary among the specific industries as to the point on the curve at which the animal is considered ready for slaughter. For poultry and pigs, for example, present practice is to slaughter near the beginning of phase three where the growth curve begins to flatten out. At this portion of the curve, the amount of time and feed required to produce a pound of gain increases, and so economics dictates that the animal should be slaughtered at that time, and replaced in the feeding facility with an animal in the second phase where weight gain is much more rapid and efficient in terms of feed conversion. For cattle however, present practice is to slaughter well into phase three. During phase 3, cattle accumulate fat, which lends palatability to meat. Presently cattle are grouped according to weight and visual clues such as frame size and breed traits. The group is then penned together and from that point each animal is substantially fed and otherwise maintained uniformly. When it is determined that the average body condition of the group is a desired body condition, all animals in the group are slaughtered.

In cattle production, for example, it is known to use ultrasound devices to measure the back fat on some live animals in an attempt to predict intramuscular fat to better judge when the desired body fat condition has been attained. While accurate measurements of back fat can be made on a live animal, back fat is known to not correlate with any degree of accuracy to intramuscular fat which is marbled through the meat, and which is accepted as adding palatability, and thus brings a premium price. Actual intramuscular fat can only be accurately assessed after the animal is slaughtered, when the animal's carcass is graded. Thus, cattle feeders are limited in the success that they can attain in providing slaughter animals that meet the desired palatability grade AAA. Presently, a feedlot operator feeds all the cattle in an attempt to most economically ensure that the maximum number achieve the most optimum grade, for example, grade AAA.

Genotype testing of feeder cattle in a typical feedlot situation by the present inventor showed a direct correlation between genotype and fat deposition. The cattle were confined in conventional pens, fed conventional rations, and slaughtered when discerned by conventional means to be market ready. The cattle were tested to determine the genotype, and were traced to the shipping point to determine the palatability grade achieved. Each pen contained a mix of unsegregated CC, CT, and TT cattle.

Results of the first test (Test 1) showed that, of 73 Hereford steers tested for genotype, 36 were CT, 37 were TT, while none were CC. The 73 cattle were When slaughtered, 48.5% of the TT carcasses graded AAA, and 19.4% of the CT carcasses graded AAA.

In Test 2, of the 50 Charolais - Angus cross steers tested for genotype, 9 were determined to be CC, 28 were CT, and 13 were TT. When slaughtered, 62% of the TT carcasses graded AAA, 29% of the CT carcasses graded AAA, and 11% of the CC carcasses graded AAA.

In Test 3, 13 Charolais cattle in each of 5 pens, or a total of 65 animals, were tested for allellotype. Of the 65 cattle, 29 were CC, 24 were CT, and 12 were TT. There was a high degree of breeding in the 65 cattle. When slaughtered, 58.3% of the TT carcasses graded AAA, 45.5% of the CT carcasses graded AAA, and 38.5% of the CC carcasses graded AAA.

The method of the present invention contemplates grouping production animals according to their genotype or, more specifically, allelotype in addition to using the phenotypic criteria currently employed in feedlot practice. For example, in one embodiment of the present invention, feedlot operators who currently group cattle according to size and frame structure, among other phenotypic traits, would group animals according to allelotype, i.e., CC, TC, or TT, which correlate with the animal's propensity to deposit fat, in order to more efficiently manage production. Thus the feeder is presented with opportunities for considerable efficiencies in livestock production.

Presently, the feeder feeds all his cattle the same, incurring the same costs for each animal, and typically, with excellent management practices, perhaps 40% will receive an optimal grade, such as AAA, and receive the premium price for the palatability grade. Of these, a significant number will have excess fat and will thus receive a reduced yield grade.

The balance of the cattle, 60%, will grade less than AAA, and thus receive a reduced price, although the feed lot costs incurred by the feeder are substantially the same for these cattle receiving the lesser grade. Grouping and feeding the cattle by genotype and , more specifically, allelotype allows the feeder to treat each group differently with a view to optimizing management strategies and increasing profit.

For example, according to one embodiment embodiment of the present invention, a group of CC cattle will have the least propensity to deposit fat, and so it could be more profitable to slaughter this group earlier in the growth curve, near the start of phase 3 where the growth curve flattens, since they have the least chance of meeting the fat requirements of the optimum or AAA grade. Such a group slaughtered early would have a very high percentage of lean carcasses, and this predictability could itself draw premiums from packers seeking to fill orders requiring lean carcasses. On the other hand, a group of TT cattle will have the most propensity to deposit fat, and so it could be more profitable to keep these on feed longer, since it is predictable that a high percentage would accumulate sufficient intramuscular fat so that the carcass would grade AAA and thus receive a premium price. Likewise, knowing that CT cattle deposit fat at an intermediate rate will allow the feed lot operator to manage this group more efficiently and profitably as well.

It is contemplated that, regardless of the desirability and premium paid for any particular body fat condition at any given time, providing the packer with a more uniform group that is predictably fat or lean will provide the feeder with the opportunity to demand and receive a premium, relative to the less uniform groups of cattle presently available. The packer will be able to buy more of the cattle with a body fat condition that he actually needs, while buying less cattle in total. The packer can thus be much better able to manage his inventory, reducing surpluses of carcasses with less desirable body fat conditions that would ordinarily be sold at a reduced price.

Thus the present invention provides a method which, in one embodiment, reduces the inventory of carcasses in beef packing operations by reducing the total number of cattle purchased in order to obtain a desired number of carcasses of a desired grade. The method comprises determining whether animals available for purchase are TT animals (i.e., homozygous with respect to the T-allele of the ob gene), CC animals (i.e., homozygous with respect to the C-allele of the ob gene), or CT animals (i.e., heterozygous with respect to the T-allele and the C-allele of the ob gene). Where the desired grade requires fat carcasses, the packer purchases TT animals, and where the desired grade requires lean carcasses, the packer purchases CC animals.

The predictability of fat deposition allows the feed lot operator to consider the premiums available for fat or lean carcasses, and tailor his decisions to maximize returns. Where production costs are high, as when feed costs are high, the feedlot operator might profit from slaughtering early. When costs are low, it might be more profitable to slaughter later. The feed lot operator can more accurately predict the particular body fat condition of a group of animals at any given point on the growth curve, and thus more effectively make decisions regarding when to slaughter any particular group.

It is also contemplated by the method of the present invention that feed rations could be tailored to more specifically achieve a desired body fat condition for each group by managing production animals' genotype generally, and, in particular, the TT/CC/CT allelotype.

Among animals of the same species and substantially the same age and weight, where other determinants of growth such as health condition and diet are equivalent, smaller framed animals will reach a stage of maturity exemplified by the start of the third phase of growth earlier than larger framed animals. Therefore, substantial leptin effects will be evidenced earlier in such smaller framed animals than in larger framed animals.

Where other determinants of growth such as health condition and diet are equivalent, a group of animals of the same species, sharing substantially the same age, weight, and frame type will attain the stage of maturity exemplified by the start of the third phase of growth at a substantially more uniform time than an otherwise equivalent group of animals, the individual members of which do not share substantially the same frame type. Therefore, where other determinants of growth are equivalent, substantial leptin effects will begin to be evidenced at a more uniform time in animals of a group segregated on the basis of frame type t han i n animals of a group not so segregated.

Importantly, grouping otherwise similar animals based on frame size is a more accurate means of achieving body condition uniformity than grouping otherwise similar animals based on body weight. When compared to large-framed animals, small-framed animals that are of substantially the same age and weight will attain the third phase of growth earlier, begin to accumulate significant amounts of body fat earlier and, thus, attain a desired body fat condition earlier. If individual animals so grouped have different ob genotypes, substantial evidence of such difference will be exhibited at substantially uniform times. Among animals sharing substantially the same weight and frame type, TT animals will accumulate fat faster during the third phase of growth than CT animals, and ob heterozygotes will accumulate fat faster during the third phase of growth than CC animals.

One embodiment of the present invention provides a method to facilitate attainment of greater efficiency in a commercial livestock feeding and finishing facility by providing a method comprising determining the genetic predisposition of each animal to deposit fat by determining ob genotype and segregating individual animals into subgroups based upon the *ob* genotype. Thus, using the method of the present invention allows an operator to produce a livestock animal group comprising a plurality of individual animals of the same species wherein a median body fat condition of the individual animals is a desired body condition and wherein actual body fat conditions of the individual animals are improvedly uniform.

The method of the present invention also provides a packer with a more uniform group .that is predictably fat or lean ensuring the feed-lot operator with the opportunity t o demand and receive a premium, relative to the less uniform groups of cattle presently available. F or example, in accordance with one embodiment of the present invention, the packer will be able to buy more cattle with a body fat condition that he actually needs, while buying less cattle in total. The packer can thus be much better able to manage his inventory, reducing surpluses of carcasses with less desirable body fat conditions that would ordinarily be sold at a reduced price. The predictability of fat deposition allows the feed lot operator to consider the premiums available for fat or lean carcasses, and tailor his decisions to maximize returns for each group. Where costs in the feedlot are high, as when feed costs are high, the operator might profit from slaughtering early. When costs are low, it might be more profitable to slaughter later. The feed lot operator, using the method of the present invention is able to more accurately predict the particular body fat condition of a group of animals at any given point on the growth curve, and thus can more effectively make decisions regarding when to slaughter any particular group.

It is also contemplated that, where demand for optimum grade, such as AAA, beef is high, feed lot operators will pay a first price for cattle homozygous with respect to the T-allele of the ob gene, and pay a second price lower than the first price for cattle heterozygous with respect to the T-allele and C-allele of the ob gene, and pay a third price lower than the second price for cattle homozygous with respect to the C-allele of the ob gene. Packers can also set premiums for cattle based upon predicted carcass grade by genotype.

The above-stated embodiments of the present invention are achieved by collecting an assembly of individual animals of substantially similar weights and frame types that have lower percentages of body fat than are required to exemplify the desired body fat condition. Prior to or upon collection of such assembly at the site of a livestock feeding facility, it is determined whether the animal is homozygous with respect to the T-allele of the ob gene, homozygous with respect to the C-allele of the ob gene, or heterozygous with respect to both T- and C-alleles.

A tissue sample containing chromosomal DNA can be collected from each individual animal to determine ob genotype. Known means c an b e u sed to disrupt animal c ells and process animal tissue samples consistent with the maintenance of chromosomal DNA integrity in such tissue samples. Standard molecular biology textbooks such as Sambrook et al. eds "Molecular Cloning: A Laboratory Manual" 2nd ed. Cold Spring Harbor Press (1989) may be consulted to design suitable protocols for the isolation of DNA samples from tissues of choice. It should be recognized, however, that the choice of a suitable tissue or sample for the isolation of DNA suitable for determining ob genotype depends upon multiple factors including the ease of obtaining the sample from the animal and the quantity of DNA present in the sample. Tissues of choice include, but are not limited to, hair, epithelial cells, blood, nasal and vaginal swabs and the like.

Each sample is processed by conventional methods such that the chromosomal DNA is purified or partially purified. The purified DNA is then assayed to distinguish the presence therein of a wild-type allele of the *ob* gene and a mutant allele of the ob gene using methods known to one skilled in the art of molecular biology. Any method for determining genotype can be used for determining the ob genotype in the present invention. Such methods include, but are not limited to, DNA sequencing, RFLP analysis, microsatellite analysis, polymerase chain reaction (PCR), ligase chain reaction (LCR), amplimer sequencing, nucleic acid hybridization, FRET-based hybridization analysis, size chromatography (e.g., capillary or gel chromatography), high throughput screening, mass spectroscopy, and fluorescence spectroscopy, all of which are well known to one of skill in the art. In particular, methods for determining nucleotide polymorphisms, particularly single nucleotide polymorphisms, are described in U.S. Patent Nos. 6,514,700; 6,503,710; 6,468,742; 6,448,407; 6,410,231; 6,383,756; 6,358,679; 6,322,980; 6,316,230; and 6,287,766 and reviewed by Chen and Sullivan, Pharmacogenomics J 2003;3(2):77-96.

One conventional means for distinguishing allelles is by mismatch PCR-RFLP. For example, as applied to an advantageous embodiment of the invention, synthetic oligonucleotide-primed amplification of the exon 2 of the ob gene followed by restriction endonuclease treatment of the amplified DNA product thereof using Kpn 2I results in a cut of the amplimer corresponding to the C allele of the ob gene, but the amplimer corresponding to the T allele is not cut. Genotyping of genotype may be carried out by testing at the intake of a feeding facility or at any time during the life of the animal and recorded, conveniently on an ear tag or the like that moves with the animal so that it is readily available.

Once the genotype is determined, individual animals are segregated into groups wherein each animal shares the same ob genotype, ie. *ob⁻* ( a TT animal), ob (a CT animal), or *ob⁺* (a CC animal), according to the method of the present invention. The animals of each group are maintained and fed together, such that the environmental, health, nutritional, and other conditions and needs of all such animals are maintained and satisfied to a substantially equivalent extent and by substantially equivalent means. Because a TT animal, exhibits an increased rate of body fat deposition compared to a C T animal, which i n turn exhibits an increased rate of body fat deposition compared to a CC animal, feedlot operators are able to treat each group differently with a view to optimizing management strategies and increasing profit.

The invention also provides a method of breeding a livestock animal with a propensity to accumulate body fat as a proportion of total body weight at a rate that is,: (i) predictable; (ii) either greater than or lesser than other livestock animals of the same species when such individual livestock animal and such other individual livestock animals are fed and maintained under conditions of substantial equivalence; and (iii) shares a substantially similar temporal time-course with animals of the same or determinably similar parentage. This object is achieved by collecting male and female livestock animals of the same species and known frame types, or germinal tissue therefrom; collecting from each above-said animal a tissue sample containing chromosomal DNA; and genotyping each tissue sample according to the means above-described, or according to equivalent means known in the art. Individual male and female livestock animals are selecting for breeding with one another based on frame type and genotype such that:
(a) large, intermediate or small frame-type progeny animals that exhibit a higher, intermediate or lower total body weight at maturity relative to each other can, with a useful degree of certainty, be predicted to be produced by mating large, intermediate, or small frame-type parental animals respectively;
(b) CC or TT or CT progeny (which can, with a useful degree of certainty, be predicted to evidence, respectively, relatively, lower, higher o r intermediate rates of body fat accumulation during the third growth phase of such progeny) can be produced by mating parental animals with known ob genotypes according known principals of inheritance; and
(c) by selecting parental animals based on frame type and ob genotype together, a multiplicity of progeny can be produced that, with a useful degree of certainty according to known principals of inheritance, can be predicted to, when fed and maintained substantially under conditions of substantial equivalence, attain a desired body fat condition with relatively greater temporal uniformity than animals selected according to existing breeding protocols.

Progeny from parental TT or CT animals will have a propensity to accumulate during growth body fat at a rate greater than the average rate of body fat accumulation by other individual livestock animals of the same species and age maintained in conditions of substantial equivalence but bred according to other protocols which would include CC animals. As the occurrence of the T-allele in the offspring increases, so will the propensity of the offspring to accumulate fat.

Furthermore, once the ob genotype of a particular progeny is known based upon the ob genotype of the parents, which can be confirmed by determining the ob genotype of the progeny, further progeny of a particular genotype can be propagated according to the methods of the invention. Thus, an additional utility of the present invention is the selective breeding for a particular ob genotype once the ob genotypes of the parents are determined, i.e., according to the principles of Mendelian genetics.

The foregoing is considered as illustrative only of the principles of the invention. Further, since numerous changes and modifications will readily occur to those skilled in the art, it is not desired to limit the invention to the exact operation shown and described, and accordingly, all such suitable changes or modifications in operation which may be resorted to are intended to fall within the scope of the claimed invention.

### REFERENCES

U.S. Patent No. 6,277,592 to Bidwell, C.A., and Spurlock, M.E. (August 21, 2001) Porcine leptin protein, nucleic acid sequences coding therefore and uses thereof.
U.S. Patent No. 6.297,027 to Spurlock, M.E. (October 2, 2001) Bovine leptin protein, nucleic acid sequences coding therefore and uses thereof.
U.S. Patent No. 6,309,853 to Friedman, J.M., Zhang, Y., and Proenca R. (October 30, 2001) Modulators of body weight, corresponding nucleic acids and proteins, and diagnostic and therapeutic uses thereof.
Barb, C.R., Yan, X., Azain, M.J., Kraeling, R.R., Rampacek, G.B., and Ramsay, T.G. (1998) Recombinant porcine leptin reduces feed intake and stimulates growth hormone secretion in swine. Domest. Anim. Endocrinol. 15: 77-86.
Bidwell, C.A., Ji, S., Frank, G.B., Cornelius, S.G., Willis, G.M., and Spurlock, M.E. (1997) Cloning and expression of the porcine obese gene. Anim. Endocrinol. 8: 191-206.
Blache, D., Tellam, R.L., Chagas, L.M., Blackbery, M.A., Vercoe, P.E., and Martin G.B. (2000) Level of nutrition affects leptin concentration in plasma and cerebrospinal fluid in sheep. J. Endrocrinol. 165: 625-637.
Buchanan, F.C., C.J. Fitzsimmons, A.G. Van Kessel, T.D. Thue, D.C. Winkelman-Sim, and S.M.Schmutz (2002) A missense mutation in the bovine leptin gene is correlated with carcass fat content and leptin mRNA levels. (in press)
Delavaud, C., Bocquier, F., Chilliard, Y., Keisler, D.H., Gertler, A., and Kann G. (2000) Plasma leptin determination in ruminants: effect of nutritional status and body fatness on plasma leptin concentration assessed by a specific RIA in sheep. J. Endocrinol. 165: 519-526.
Ehrhardt, R.A., Slepetis, RM., Siegal-Willott, J., Van Amburgh, M.E., Bell, A.W., and Boiselair, Y.R. (2000) Development of a specific radioimmunoassay to measure physiological changes of circulating leptin in cattle and sheep. J. Endocrinol. 166: 519-528.
Fitzsimmons, C.J., Schmutz, S.M., Bergen, R.D., and McKinnon J.J. (1998) A potential association between the BM 1500 microsatellite and fat deposition in beef cattle. Mammalian Genome 9: 432-434 (1998).
Freidman, J.M. and Leibel, R.L (1992) Tackling a weights problem. Cell 69: 217-220.
Halaas, J.L., Gajiwala, K.S., Maffie, M., Cohen, S.L., Chait, B.T., Rabinowitz, D., Lallone, R.L., Burley, S.K., and Freidman, J.M. (1995) Weight-reducing effects of the plasma protein encoded by the obese gene. Science 269: 543-546.
Henry, B.A., Goding, J.W., Alexander, W.S., Tilbrook, A.J., Canny, B.J., Dunshea, F., Rao, A., Mansell, A., and Clarke, I.J. (1999) Central administration of leptin to ovariectomized ewes inhibits food intake without affecting the secretion of hormones from the pituitary gland: evidence for a dissociation of effects on appetite and neuroendocrine function. 140: 1175-1182.
Isse, N., Ogawa, Y., Tamura, N., Maxuzaki, H., Mori K. et al (1995) Structural organization and chromosomal assignment of the human obese gene. J. Bio. Chem. 270: 27728-27733.
Kennes, Y.M., Murphy, B.D., Pothier, F., and Palin, M.-F. (2001) Characterization of swine lepin (LEP) polymorphisms and their association with productin traits. Anim. Genetics 32: 215-218.
Kulig, H., Grzesiak, W., and Szatkowska, L (2001) Effect of leptin gene polymorphism on growth and carcass traits in pigs. Arch. Tierz. Dummorscorf 44: 291-296.
Neuenschwander, S., Rettenberger, G., Meijerink E. Jörg, H, and Stranzinger, G. (1996) Partial characterization of porcine obesity gene (OBS) and its localization to chromosome 18 by somatic cell hybrids Anim. Genet. 27: 275-278.
Ramsay, T.G., Yan, X., and Morrison, C. (1998) The obesity gene in swine: sequence and expression of porcine leptin. J. Anim. Sci. 76: 484-490.
Raver, N., Taouis, M., Dridi, S., Derouet, M., Simon, J., Robinzon, B., Djiane J., and Gertler, A. (1998) Large-scale preparation of biologically active recombinant chicken obese protein (leptin). Protein Expression and Purification 14: 403-408.
Robert, C., Palin, M.-F., Coulombe, N., Roberge, C., Silversides, F.G., Benkel, B.F., McKay, R.M., and Pelletier, G. (1998) Backfat thickness in pigs is positively associated with leptin mRNA levels. Can. J. Anim. Sci. 78: 473-482.
Sambrook et al. eds., (1989) "Molecular Cloning: A Laboratory Manual" 2nd ed. Cold Spring Harbor Press.
Saskai, S., Clutter, A.C., and Barendse, W. (1996) Assignment of the porcine obese (leptin) gene to Chromosome 18 by linkage analysis of a new PCR-based polymorphism. Mamm. Genome 7: 471-471.
Stone, RT., Kappes, S.M., and Beattie, C.W. (1996) The bovine monologue of the obese gene maps to chromosome 4. Mamm. Benome 7: 399-400.
Zhang, Y., Proenca, R., Maffei, M., Barone M., Leopold, L. and Friedman, J.M. (1994) Positional cloning of the mouse obese gene and its human homologue. Nature 372: 425-432

## Claims

1. A method for producing livestock animal sub-groups of the same species, from a group of livestock animals of the same species comprising the sub-groups, wherein the animals of each sub-group have similar body fat predispositions, comprising:
(a) determining genetic predisposition of each animal to deposit fat by determining ob genotype; and
(b) segregating individual animals into the sub-groups based upon the *ob* genotype; wherein determining ob genotype comprises detecting an ob gene polymorphism, wherein the ob gene polymorphism is a single nucleotide polymorphism and wherein determining comprises determining whether the animal is a TT animal homozygous with respect to the T-allele of the *ob* gene, a CC animal homozygous with respect to the C-allele of the *ob* gene, or a CT animal heterozygous with respect to the T-allele and the C-allele of the *ob* gene.

2. The method of claim 1 further comprising collecting an assembly of individual animals of similar frame type and weight, the median body fat condition of which is divergent from the desired body condition and which divergence is exemplified by lesser amounts of body fat, including intramuscular fat and back fat in such individual animals than is desired in an animal having the desired body condition.

3. The method of claim 1 further comprising maintaining the animals of the sub-group together and feeding such animals until the median body fat condition of individual animals of the sub-group is of the desired body fat condition..

4. The method of claim 1 wherein segregating comprises segregating the individual animals into at least one sub-group wherein animals of the sub-group are:
(i) TT animals homozygous with respect to the T-allele of the ob gene;
(ii) CC animals homozygous with respect to the C-allele of the ob gene; or
(iii) CT animals heterozygous with respect to the T-allele and the C-allele of the ob gene.

5. The method of claim 4 further comprising segregating the individual animals into three sub-groups wherein: (i) animals of a first sub-group are TT animals homozygous with respect to the T-allele of the ob gene; (ii) animals of a second sub-group are CC animals homozygous with respect to the C-allele of the ob gene; and (iii) animals of a third sub-group are CT animals heterozygous with respect to the T-allele and the C-allele of the ob gene; and
(a) maintaining animals of the first sub-group together and separate from animals of other sub-groups, and feeding the animals in the first sub-group uniformly until the median body fat condition of individual animals of the first sub-group is a first desired body fat condition;
(b) maintaining animals of the second sub-group together and separate from animals of other sub-groups, and feeding the animals in the second sub-group uniformly until the median body fat condition of individual animals of the second sub-group is a second desired body fat condition; and
(c) maintaining animals of the third sub-group together and separate from animals of other sub-groups, and feeding the animals in the third sub-group uniformly until the median body fat condition of individual animals of the third sub-group is a third desired body fat condition.

6. The method of claim 5 wherein the first, second, and third desired body fat conditions are the same.

7. A method of producing a progeny livestock animal with a predictable propensity to accumulate body fat during growth comprising:
(a) determining genetic predisposition of potentially parental male and potentially parental female livestock, or germinal material thereof, by determining ob genotype; and
(b) selectively breeding individuals from among potentially parental male and potentially parental female livestock animals, or germinal material thereof, based on *ob* genotype;
thereby obtaining a progeny livestock animal with a predictable propensity to accumulate body fat during growth; wherein determining ob genotype comprises detecting an ob gene polymorphism, wherein the ob gene polymorphism is a single nucleotide polymorphism and wherein determining genetic predisposition comprises determining whether the animal is a TT animal homozygous with respect to the T-allele of the ob gene, a CC animal homozygous with respect to the C-allele of the *ob* gene, or a CT animal heterozygous with respect to the T-allele and the C-allele of the *ob* gene.

8. The method of claim 7 further comprising collecting potentially parental male and potentially parental female livestock animals of the same species, or germinal material thereof, to permit propagation of progeny.

9. The method of claim 7 wherein selectively breeding comprises (i) producing a progeny livestock animal, with a first propensity to accumulate body fat during growth, by selectively breeding potentially parental male and potentially parental female livestock animals wherein at least one of the potentially parental livestock animals is a TT animal and the other of the parental animals is either a TT animal homozygous with respect to the mutant allele of the *ob* gene or a CT animal heterozygous with respect to the T-allele and the C-allele of the *ob* gene; or (ii) producing a progeny livestock animal, with a second propensity to accumulate body fat during growth, by selectively breeding potentially parental male and potentially parental female livestock animals wherein at least one of the potentially parental livestock animals is a CC animal and the other of the parental animals is either a CC animal homozygous with respect to the wild type allele of the *ob* gene or a CT animal heterozygous with respect to the T-allele and the C-allele of the *ob* gene.

10. The method of claim 9 wherein the first propensity to accumulate body fat during growth is a greater propensity than the second propensity to accumulate body fat during growth.

11. A method of producing by breeding an individual livestock animal with a genotype to accumulate body fat during growth and a phenotype of a predictable frame type at maturity, comprising:
(a) determining the genotype of a potentially parental male and a potentially parental female livestock, or germinal material thereof, by determining *ob* genotype thereof;
(b) determining the phenotype for a predictable frame type of each potentially parental male and potentially parental female livestock animal; and
(c) breeding individuals among the potentially parental male and potentially parental female livestock animals to select for an individual livestock animal with a genotype to accumulate body fat during growth and a phenotype of a desired frame type;
thereby obtaining an individual livestock animal with a genotype to accumulate body fat during growth and a phenotype of a predictable frame type at maturity; wherein determining ob genotype comprises detecting an ob gene polymorphism, wherein the ob gene polymorphism is a single nucleotide polymorphism and wherein determining genetic predisposition comprises determining whether the animal is a TT animal homozygous with respect to the T-allele of the ob gene, a CC animal homozygous with respect to the C-allele of the ob gene, or a CT animal heterozygous with respect to the T-allele and the C-allele of the *ob* gene.

12. The method of any one of claims 1 to 11 wherein the livestock animal species is swine.

13. The method of any one of claims 1 to 11 wherein the livestock animal species is a cow.

## Patentansprüche

1. Verfahren zur Erzeugung von Viehuntergruppen der gleichen Art aus einer Gruppe von Vieh der gleichen Art, weiche die Untergruppen umfaßt, wobei die Tiere jeder Untergruppe ähnliche Körperfett-Prädispositionen aufweisen, bei dem man:
a) die genetische Prädisposition jedes Tieres, Fett einzulagern, anhand der Ermittlung des *ob*-Genotyps ermittelt und
b) die individuellen Tiere auf der Grundlage des *ob*-Genotyps in die Untergruppen einteilt, wobei die Ermittlung des *ob*-Genotyps die Erfassung eines ob-Genpolymorphismus umfaßt, wobei der ob-Genpolymorphismus ein Einzelnukleotid-Polymorphismus ist, und wobei die Ermittlung umfaßt, daß man ermittelt, ob das Tier ein TT-Tier ist, das im Hinblick auf das T-Allel des *ob*-Gens homozygot ist, ein CC-Tier ist, das im Hinblick auf das C-Allel des *ob*-Gens homozygot ist, oder ein CT-Tier ist, das im Hinblick auf das T-Allel und das C-Allel des *ob*-Gens heterozygot ist.

2. Verfahren nach Anspruch 1, bei dem man weiterhin eine Gruppe von individuellen Tieren mit ähnlichem Körperbau und Gewicht erfaßt, deren mittlerer Körperfettzustand von dem gewünschten Körperzustand abweicht, wobei sich die Abweichung durch geringere Mengen an Körperfett, einschließlich intramuskuläres Fett und Rükkenfett, bei diesen individuellen Tieren veranschaulicht als bei einem Tier gewünscht ist, welches den gewünschten Körperzustand aufweist.

3. Verfahren nach Anspruch 1, bei dem man weiterhin die Tiere der Untergruppe zusammenhält und diese Tiere füttert, bis der mittlere Körperfettzustand von individuellen Tieren der Untergruppe der gewünschte Körperfettzustand ist.

4. Verfahren nach Anspruch 1, wobei das Einteilen das Einteilen der individuellen Tiere auf wenigstens eine Untergruppe umfaßt, wobei die Tiere der Untergruppe:
(i) TT-Tiere sind, die im Hinblick auf das T-Allel des *ob*-Gens homozygot sind,
(ii) CC-Tiere sind, die im Hinblick auf das C-Allel des *ob*-Gens homozygot sind, oder
(iii) CT-Tiere sind, die im Hinblick auf das T-Allel und das C-Allel des *ob*-Gens heterozygot sind.

5. Verfahren nach Anspruch 4, bei dem man weiterhin die individuellen Tiere in drei Untergruppen einteilt, wobei: (i) die Tiere einer ersten Untergruppe TT-Tiere sind, die im Hinblick auf das T-Allel des *ob*-Gens homozygot sind, (ii) die Tiere einer zweiten Untergruppe CC-Tiere sind, die im Hinblick auf das C-Allel des ob-Gens homozygot sind, und (iii) die Tiere einer dritten Untergruppe CT-Tiere sind, die im Hinblick auf das T-Allel und das C-Allel des *ob*-Gens heterozygot sind, und wobei man
a) die Tiere der ersten Untergruppe gemeinsam und separat von den Tieren von anderen Untergruppen hält und die Tiere in der ersten Untergruppe einheitlich füttert, bis der mittlere Körperfettzustand von individuellen Tieren der ersten Untergruppe ein erster gewünschter Körperfettzustand ist,
b) die Tiere der zweiten Untergruppe gemeinsam und separat von den Tieren von anderen Untergruppen hält und die Tiere in der zweiten Untergruppe einheitlich füttert, bis der mittlere Körperfettzustand von individuellen Tieren der zweiten Untergruppe ein zweiter gewünschter Körperfettzustand ist, und
c) die Tiere der dritten Untergruppe gemeinsam und separat von den Tieren von anderen Untergruppen hält und die Tiere in der dritten Untergruppe einheitlich füttert, bis der mittlere Körperfettzustand von individuellen Tieren der dritten Untergruppe ein dritter gewünschter Körperfettzustand ist.

6. Verfahren nach Anspruch 5, wobei die ersten, zweiten und dritten gewünschten Körperfettzustände gleich sind.

7. Verfahren zur Erzeugung einer Viehnachkommenschaft mit einer vorhersagbaren Neigung, Körperfett während des Wachstums einzulagern, bei dem man:
a) die genetische Prädisposition von potentiellem männlichen Elternvieh und potentiellem weiblichen Elternvieh oder des Keimmaterials davon durch Ermitteln des *ob*-Genotyps ermittelt und
b) auf der Grundlage des ob-Genotyps selektiv Individuen zwischen potentiellem männlichen Elternvieh und potentiellem weiblichen Elternvieh oder aus Keimmaterial davon züchtet,
wodurch eine Viehnachkommenschaft mit einer vorhersagbaren Neigung zur Einlagerung von Körperfett während des Wachstums erhalten wird, wobei die Ermittlung des ob-Genotyps die Erfassung eines ob-Genpolymorphismus umfaßt, wobei der *ob-*Genpolymorphismus ein Einzelnukleotid-Polymorphismus ist, und wobei das Ermitteln der genetischen Prädisposition umfaßt, daß man ermittelt, ob das Tier ein TT-Tier ist, das im Hinblick auf das T-Allel des *ob*-Gens homozygot ist, ein CC-Tier ist, das im Hinblick auf das C-Allel des *ob*-Gens homozygot ist, oder ein CT-Tier ist, das im Hinblick auf das T-Allel und das C-Allel des ob-Gens heterozygot ist.

8. Verfahren nach Anspruch 7, welches weiterhin umfaßt, daß man potentielles männliches Elternvieh und potentielles weibliches Elternvieh der gleichen Art oder das Keimmaterial davon zusammenbringt, um die Propagierung von Nachkommenschaft zu ermöglichen.

9. Verfahren nach Anspruch 7, wobei das selektive Züchten umfaßt, daß man (i) eine Viehnachkommenschaft mit einer ersten Neigung, Körperfett während des Wachstums einzulagern, erzeugt, indem man selektiv potentiell männliches Elternvieh und potentiell weibliches Elternvieh züchtet, wobei wenigstens ein Tier unter dem potentiellen Elternvieh ein TT-Tier ist und das/die andere/n Elterntier/e entweder (ein) TT-Tier(e) ist/sind, das/die im Hinblick auf das mutierte Allel des *ob*-Gens homozygot ist, oder (ein) CT-Tier(e) ist/sind, das/die im Hinblick auf das T-Allel und das C-Allel des *ob*-Gens heterozygot ist/sind, oder (ii) eine Viehnachkommenschaft mit einer zweiten Neigung, Körperfett während des Wachstums einzulagern, erzeugt, indem man selektiv potentielles männliches Elternvieh und potentielles weibliches Elternvieh züchtet, wobei wenigstens ein Tier unter dem potentiellen Elternvieh ein CC-Tier ist und das/die andere/n Elterntier(e) entweder (ein) CC-Tier(e) ist/sind, die im Hinblick auf das Wildtypallel des *ob*-Gens homozygot ist/sind, oder (ein) CT-Tier(e) ist/sind das/die im Hinblick auf das T-Allel und das C-Allel des ob-Gens heterozygot ist/sind.

10. Verfahren nach Anspruch 9, wobei die erste Neigung, Körperfett während des Wachstums einzulagern, eine größere Neigung ist als die zweite Neigung, Körperfett während des Wachstums einzulagern.

11. Herstellungsverfahren, bei dem man ein individuelles Stück Vieh mit einem Genotyp für das Einlagern von Körperfett während des Wachstums und mit einem Phänotyp eines vorhersagbaren Körperbaus bei Reife züchtet, wobei man:
(a) den Genotyp von einem potentiellen männlichen Elternvieh und einem potentiellen weiblichen Elternvieh oder von deren Keimmaterial ermittelt, indem man den *ob*-Genotyp davon ermittelt,
(b) den Phänotyp für einen vorhersagbaren Körperbau von jedem potentiellen männlichen Elternvieh und jedem potentiellen weiblichen Elternvieh ermittelt, und
(c) Individuen zwischen dem potentiellen männlichen Elternvieh und dem potentiellen weiblichen Elternvieh züchtet, um auf ein individuelles Vieh mit einem Genotyp zum Einlagern von Körperfett während des Wachstums und mit einem Phänotyp eines gewünschten Körperbaus zu selektionieren,
wodurch ein individuelles Vieh mit einem Genotyp zum Einlagern von Körperfett während des Wachstums und mit einem Phänotyp eines vorhersagbaren Körperbaus bei Reife erhalten wird, wobei das Ermitteln des *ob*-Genotyps umfaßt, daß man einen ob-Genpolymorphismus erfaßt, wobei der ob-Genpolymorphismus ein Einzelnukleotid-Polymorphismus ist, und wobei das Ermitteln der genetischen Prädisposition umfaßt, daß man ermittelt, ob das Tier ein TT-Tier ist, das im Hinblick auf das T-Allel des *ob-*Gens homozygot ist, ein CC-Tier ist, das im Hinblick auf das C-Allel des *ob*-Gens homozygot ist, oder ein CT-Tier ist, das im Hinblick auf das T-Allel und das C-Allel des *ob*-Gens heterozygot ist.

12. Verfahren nach einem der Ansprüche 1 bis 11, wobei die Viehart das Schwein ist.

13. Verfahren nach einem der Ansprüche 1 bis 11, wobei die Viehart das Rind ist.

## Revendications

1. Procédé pour la production de sous-groupes d'animaux servant de bétail, de la même espèce, à partir d'un groupe d'animaux servant de bétail de la même espèce comprenant les sous-groupes, dans lequel les animaux de chaque sous-groupe ont des prédispositions similaires concernant les graisses corporelles, comprenant :
(a) la détermination de la prédisposition génétique de chaque animal au dépôt des graisses en déterminant le génotype ob ; et
(b) la ségrégation des animaux individuels en les sous-groupes d'après le génotype *ob* ; dans lequel la détermination du génotype *ob* comprend la détection d'un polymorphisme du gène *ob*, le polymorphisme du gène ob étant un polymorphisme de nucléotide unique, et la détermination comprenant l'étape consistant à déterminer si l'animal est un animal TT homozygote par rapport à l'allèle T du gène ob, un animal CC homozygote par rapport à l'allèle C du gène ob, ou un animal CT hétérozygote par rapport à l'allèle T et l'allèle C du gène ob.

2. Procédé suivant la revendication 1, comprenant en outre la réunion d'un ensemble d'animaux individuels ayant un type de carcasse et un poids similaires, dont la condition de graisses corporelles moyenne diverge de la condition corporelle désirée, divergence qui est illustrée par de moindres quantités de graisses corporelles, comprenant la graisse intramusculaire et la graisse dorsale chez ces animaux individuels, par rapport aux valeurs désirées chez un animal ayant la condition corporelle désirée.

3. Procédé suivant la revendication 1, comprenant en outre l'étape consistant à maintenir ensemble les animaux du sous-groupe et à nourrir ces animaux jusqu'à ce que la condition de graisses corporelles moyenne des animaux individuels du sous-groupe corresponde à la condition de graisses corporelles désirée.

4. Procédé suivant la revendication 1, dans lequel la ségrégation comprend la ségrégation des animaux individuels en au moins un sous-groupe, où les animaux du sous-groupe sont :
(i) des animaux TT homozygotes par rapport à l'allèle T du gène ob ;
(ii) des animaux CC homozygotes par rapport l'allèle C du gène ob ; ou
(iii) des animaux CT hétérozygotes par rapport à l'allèle T et à l'allèle C du gène ob.

5. Procédé suivant la revendication 4, comprenant en outre la ségrégation des animaux individuels en trois sous-groupes dans lesquels : (i) les animaux d'un premier sous-groupe sont des animaux TT homozygotes par rapport à l'allèle T du gène *ob* ; (ii) les animaux d'un deuxième sous-groupe sont des animaux CC homozygotes par rapport à l'allèle C du gène *ob* ; et (iii) les animaux d'un troisième sous-groupe sont des animaux CT hétérozygotes par rapport à l'allèle T et à l'allèle C du gène ob ; et
(a) le maintien des animaux du premier sous-groupe ensemble et à l'état séparé des animaux des autres sous-groupes, et l'alimentation des animaux dans le premier sous-groupe uniformément jusqu'à ce que la condition de graisses corporelles moyenne des animaux individuels du premier sous-groupe soit une première condition de graisses corporelles désirée ;
(b) le maintien des animaux du deuxième sous-groupe ensemble et à l'état séparé des animaux des autres sous-groupes, et l'alimentation des animaux dans le deuxième sous-groupe uniformément jusqu'à ce que la condition de graisses corporelles moyenne des animaux individuels du deuxième sous-groupe soit une condition de graisses corporelles désirée ; et
(c) le maintien des animaux du troisième sous-groupe ensemble et à l'état séparé des animaux des autres sous-groupes, et l'alimentation des animaux du troisième sous-groupe uniformément jusqu'à ce que la condition de graisses corporelles moyenne des animaux individuels du troisième sous-groupe soit une troisième condition de graisses corporelles désirée.

6. Procédé suivant la revendication 5, dans lequel les première, deuxième et troisième conditions de graisses corporelles désirées sont identiques.

7. Procédé pour la production d'un animal de la descendance, servant de bétail, ayant une tendance prévisible à l'accumulation des graisses corporelles au cours de la croissance, comprenant :
(a) la détermination de la prédisposition génétique du bétail mâle potentiellement parentéral et du bétail femelle potentiellement parental, ou de leur matériel germinal, en déterminant le génotype ob ; et
(b) la reproduction sélective d'individus provenant des animaux servant de bétail mâles potentiellement parentaux et femelles potentiellement parentaux, ou de leurs matières germinales, d'après le génotype *ob* ;
ce qui permet d'obtenir un animal de la descendance, servant de bétail, ayant une tendance prévisible à l'accumulation des graisses corporelles au cours de la croissance ; dans lequel la détermination du génotype ob comprend la détection d'un polymorphisme du gène ob, le polymorphisme du gène ob étant un polymorphisme de nucléotide unique, et la détermination de la prédisposition génétique comprend l'étape consistant à déterminer si l'animal est un animal TT homozygote par rapport à l'allèle T du gène ob, un animal CC homozygote par rapport à l'allèle C du gène ob ou un animal CT hétérozygote par rapport à l'allèle T et à l'allèle C du gène ob.

8. Procédé suivant la revendication 7, comprenant en outre la réunion des animaux servant de bétail mâles potentiellement parentaux et femelles potentiellement parentaux de la même espèce, ou de leur matériel germinal, pour permettre la multiplication de la descendance.

9. Procédé suivant la revendication 7, dans lequel la reproduction de manière sélective comprend (i) la production d'un animal servant de bétail de la descendance, ayant une première tendance à l'accumulation des graisses corporelles au cours de la croissance, la reproduction de manière sélective des animaux servant de bétail mâles potentiellement parentaux et femelles potentiellement parentaux, au moins l'un des animaux servant de bétail potentiellement parentaux étant un animal TT et l'autre des animaux parentaux étant un animal TT homozygote par rapport à l'allèle mutant du gène ob ou un animal CT hétérozygote par rapport l'allèle T et à l'allèle C du gène *ob ;* ou (ii) la production d'un animal servant de bétail de la descendance, ayant une seconde tendance à l'accumulation des graisses corporelles au cours de la croissance, par reproduction de manière sélective des animaux servant de bétail mâles potentiellement parentaux et femelles potentiellement parentaux, où au moins l'un des animaux servant de bétail potentiellement parentaux est un animal CC et l'autre des animaux parentaux est un animal CC homozygote par rapport à l'allèle de type sauvage du gène ob ou un animal CT hétérozygote par rapport à l'allèle T et à l'allèle C du gène ob.

10. Procédé suivant la revendication 9, dans lequel la première tendance à l'accumulation des graisses corporelles au cours de la croissance est une tendance supérieure à la seconde tendance à l'accumulation des graisses corporelles au cours de la croissance.

11. Procédé pour produire, par reproduction, un animal individuel servant de bétail ayant un génotype d'accumulation des graisses corporelles au cours de la croissance et un phénotype d'un type de carcasse prévisible à la maturité, comprenant :
(a) la détermination du génotype d'un animal servant de bétail mâle potentiellement parental et d'un animal servant de bétail femelle potentiellement parental, ou de leur matériel germinal, en déterminant leur génotype ob ;
(b) la détermination du phénotype d'un type de carcasse prévisible de chacun des animaux servant de bétail mâle potentiellement parental et femelle potentiellement parental ; et
(c) la reproduction d'individus parmi les animaux servant de bétail mâles potentiellement parentaux et femelles potentiellement parentaux pour sélectionner un animal individuel servant de bétail ayant un génotype d'accumulation des graisses corporelles au cours de la croissance et un phénotype d'un type de carcasse désirée ;
ce qui permet d'obtenir un animal individuel servant de bétail ayant un génotype d'accumulation des graisses corporelles et un phénotype de type de carcasse prévisible à la maturité ; la détermination du génotype ob comprenant la détection d'un polymorphisme du gène ob, le polymorphisme du gène ob étant un polymorphisme de nucléotide unique, et la détermination de la prédisposition génétique comprenant l'étape consistant à déterminer si l'animal est un animal TT homozygote par rapport à l'allèle T du gène ob, un animal CC homozygote par rapport à l'allèle C du gène ob ou un animal CT hétérozygote par rapport à l'allèle T et à l'allèle C du gène *ob.*

12. Procédé suivant l'une quelconque des revendications 1 à 11, dans lequel l'espèce animale servant de bétail est le porc.

13. Procédé suivant l'une quelconque des revendications 1 à 11, dans lequel l'espèce animale servant de bétail est une vache.
